# EUROPEAN PATENT APPLICATION

(11) **EP 4 785 925 A1**
(43) Date of publication of application: **05.08.2026**
(21) Application number: 25154605.7
(22) Date of filing: 29.01.2025
(51) Int. Cl.: A61K 8/34, A61K 8/44, A61K 8/46, A61K 8/73, A61Q 11/00

(54) **NON-AQUEOUS DENTIFRICE COMPRISING HYALURONIC ACID**

(71) Applicant: Haleon UK IP Limited, Weybridge, Surrey KT13 0NY (GB)
(72) Inventor: The designation of the inventor has not yet been filed
(74) Representative: Haleon Patent Department

(57) **Abstract**

The present invention relates to a non-aqueous dentifrice composition. In particular the invention relates to a non-aqueous dentifrice composition comprising a combination of surfactants i.e. a betaine and a taurate surfactant; or a betaine and an alkyl sulphate surfactant; or a betaine, a taurate and an alkyl sulphate surfactant in combination with hyaluronic acid or salt thereof. Such a dentifrice composition demonstrates pleasant organolpetic properties and foaming properties and is of use in oral care.

## Description

### FIELD OF THE INVENTION

The present invention relates to a non-aqueous dentifrice composition. In particular the invention relates to a non-aqueous dentifrice composition comprising a solvent, a combination of surfactants, a carboxyvinyl polymer and with hyalauronic acid or a salt thereof. Such a dentifrice composition demonstrates pleasant organolpetic properties and is of use in oral care.

### BACKGROUND OF THE INVENTION

There are many dentifrice additives which are incompatible with aqueous systems of typical dentifrice formulations. One way of overcoming this incompatibility problem is to formulate such additives in a non-aqueous (anhydrous) formulation. However in addition to circumventing the water incompatibility problem, such formulations must also be successful from a consumer acceptance standpoint and demonstrate, for example, acceptable taste, consistency and adequate foaming on brushing of teeth.

US 3,574,824 (Warner-Lambert) describes an anhydrous toothpaste base comprising *inter alia* an oil, a combination of polyethylene glycols and a non-toxic, non-ionic emulsifier which is a mixture of glycerides. US 3, 574,824 discloses that an experimental paste was made using oily liquids, such as vegetable oil or extra light mineral oil, and a suitable surfactant. However when the paste was brought into contact with water during brushing there was insufficient foaming in the oral cavity. This was thought to be due to the fact that the foaming agent (sodium lauryl sulphate) was "hindered" in the anhydrous mass and was unable to concentrate at the air-water interface during brushing to form air bubbles or foam.

US 4,647,451 (Colgate-Palmolive Company) describes anhydrous dentifrice compositions having desirable rheological, sensory and hygienic characteristics containing a polysaccharide gum and glycerine humectant. According to US 4,647,451, such compositions comprise organic surface active agents which may be anionic, non-ionic, ampholytic, or cationic in nature. Compositions exemplified therein contain sodium lauryl sulphate as the sole surfactant.

US 5,670,137 (L'Oreal) describes a dentifrice composition containing an anhydrous medium comprising glycerine, at least one hydroxyethylcellulose, at least one pyrogenetic silica. According to US 5,670,137 such a composition also contains one or more foaming surfactants which may be anionic, amphoteric, zwitterionic, cationic or non-ionic. Compositions exemplified therein contain sodium lauryl sulphate as the sole surfactant.

WO96/03108 (SmithKline Beecham plc) and WO 2002/038119 (SmithKline Beecham plc) both describe non-aqueous dentifrice compositions. Anionic, cationic, non-ionic and amphoteric surfactants are disclosed for use as suitable surfactants. A particularly preferred anionic surfactant is identified as sodium methyl cocyl taurate, marketed under the name "Adinol CT 95", this being the sole surfactant exemplified for use in the compositions therein.

WO2005/063185 (Novamin Technology Inc.) describes non-aqueous compositions of bioactive glass particulates in a non-aqueous carrier of the type disclosed in US Patent No. 5,882,630 (Gates). US 5,882,630 corresponds to the US patent derived from WO96/03108, discussed above. According to WO2005/063185, compositions therein may optionally comprise agents conventionally used in dentifrice formulations including, for example, a foaming agent such as sodium lauryl sulphate. Compositions exemplified therein contain sodium lauryl sulphate as the sole surfactant.

WO2010/115037 (Colgate-Palmolive Company) describes non-aqueous compositions comprising carrageenan or a carboxymethylcellulose gum, a humectant and a bioacceptable and bioactive glass. According to WO2010/1155037, compositions therein may comprise additives conventionally used in dentifrice compositions including for example a surfactant such as sodium lauryl sulphate. Compositions exemplified therein contain sodium lauryl sulphate as the sole surfactant.

Whilst the non-aqueous compositions described in the prior art address some of the problems encountered with formulating dentifrice additives that are incompatible with aqueous-based systems, there nevertheless remains a need for alternatives. Ideally such alternative compositions should further demonstrate one or more properties that are key drivers of consumer acceptance including, for example, having consumer-acceptable organolpetic properties. Ideally the organolepic properties of such a composition will be at least as good as or preferably improved over those seen in comparable, marketed non-aqueous dentifrice products.

It is an object of the invention to provide such a composition.

### SUMMARY OF THE INVENTION

In one aspect the invention provides A non-aqueous dentifrice composition comprising;
a) a solvent, wherein the solvent is selected from the group comprising glycerin, sorbitol, propylene glycol, polyethylene glycol and mixtures thereof.
b) a carboxyvinyl polymer wherein the carboxyvinyl polymer is present in the range from 0. 1 to 7.5% by weight of composition;
c) a surfactant system consisting of a first surfactant which is a betaine in combination with a second surfactant which is a taurate or a C₁₀₋₂₀ alkyl sulphate or mixtures thereof; and
d) hyaluronic acid or a salt thereof.

Surprisingly it has been discovered that a non-aqueous composition comprising a solvent, a carboxyvinyl polymer, a surfactant system consisting of a combination of surfactants, specifically a betaine and a taurate, or a betaine and an C₁₀₋₂₀ alkyl sulphate, in combination with a hyaluronic acid or a salt thereof provides significantly superior organoleptic properties compared to that observed for the composition without the hyalauronic acid. Such superior organoleptic properties include one or more of improved mouthfeel, improved foam dispersion and consistency, and improved foam characteristics such as intensity and density, all of which are desired attributes in a dentifrice composition. In some embodiments foam characteristics are improved without modifying significantly on taste properties.

### DETAILED DESCRIPTION OF THE INVENTION

As used herein, the term "non-aqueous" means anhydrous or substantially free of water. The individual components of the non-aqueous composition may contain limited amounts of water as long as the overall composition remains substantially free of water.

As used herein the term "dentifrice" includes any semi-solid preparation in the form of a paste, cream or gel for use in cleaning all or a portion of the oral cavity of an individual.

As used herein the term "oral cavity" means an individual's teeth and gums including all periodontal regions including teeth down to the gingival margins and/or the periodontal pockets.

The composition requires a solvent, wherein the solvent comprises glycerin, sorbitol, propylene glycol, polyethylene glycol or mixtures thereof.

In one embodiment the solvent comprises glycerin. It is well known that commercially available glycerin may contain between 0.1-2.0% by weight of water which is in association with the glycerin. Typically this amount is < 0.5% for example between 0.1-0.5% by weight of the glycerin. This small amount of water is bound to the glycerin and is therefore not available to the other ingredients. The skilled person would still consider a composition containing glycerin as being non-aqueous. The solvent should in any case be as anhydrous as possible.

In one embodiment the solvent comprises polyethylene glycol. Suitably, the polyethylene glycol will be selected from PEG 300, PEG 400 and mixtures thereof. In one embodiment the polyethylene glycol comprises PEG 400.

In one embodiment the solvent comprises a mixture of glycerin and polyethylene glycol.

The formulation solvent is used to make the formulation up to 100 %, and suitably the total amount of solvent may be present in the range of from about 20 % to about 95 % by weight of the non-aqueous composition.

Suitably the solvent comprises glycerin present from about 35 to about 75%. In one embodiment the glycerin is present from about 50 to about 70% by weight of the non-aqueous composition.

Suitably the solvent comprises polyethylene glycol present from about 0.1% to about 40% by weight of the non aqueous composition. In one embodiment the polyethylene glycol is present from about 15 to about 25% by weight of the non-aqueous composition.

The composition of the present invention comprises a carboxyvinyl polymer such as a carbomer as a thickening agent. The carboxyvinyl polymer is present in the range of from about 0.1 to about 7.5 % by weight of the non-aqueous dentitrice composition.

The carboxy vinyl polymer is present in the formulation to give the product a rheology closer to that of a conventional dentifrice.

A carbomer comprises synthetic high molecular-weight cross-linked polymers of acrylic acid. The polymer chains formed of repeating units of acrylic acid may be cross-linked with, for example: allyl sucrose to provide a carbomer available commercially in one form as Carbopol^{™} 934; ethers of pentaerythritol to provide a carbomer available commercially in one form as Carbopol^{™} 974; or with divinyl glycol , available commercially in one form as Noveon^{™} AA-1. Carbopol^{™} polymers are manufactured by B.F. Goodrich Company. In one embodiment the carboxyvinyl polymer comprises Carbopol^{™} 974.

In one embodiment the carboxyvinyl polymer is present in an amount from about 0.3 to about 1.0% by weight of the composition.

In order to produce a composition that is smooth and does not show any signs of stickiness, use of a particular ratio of carboxyvinyl polymer to polyethylene glycol is desirable.

Advantageously, the ratio of carboxyvinyl polymer to polyethylene glycol is in the range of about 1:15 to about 1:30.

The composition according to the invention comprises a surfactant system. The surfactant system consists of a first surfactant and a second surfactant. In certain embodiments the surfactant system consists of a first surfactant and a second surfactant wherein the second surfactant consists of a mixture of surfactants.

A first surfactant for use in the surfactant system of a composition according to the invention belongs to the class of compounds known as betaines. Structurally, betaine compounds contain an anionic functional group such as a carboxylate functional group and a cationic functional group such as quaternary nitrogen functional group separated by a methylene moiety. They include n-alkyl betaines such as cetyl betaine and behenyl betaine, and n-alkylamido betaines such as cocoamidopropyl betaine. In one embodiment the betaine is cocoamidopropyl betaine, commercially available under the trade name Tego Betain. Suitably the betaine is present in an amount ranging from about 0.05 to about 4% by weight of the non-aqueous composition, for example from about 0.2 to about 2.0% by weight of the non-aqueous composition.

A second surfactant for use in the surfactant system of a composition according to the invention is selected from a taurate or a C₁₀₋₂₀ alkyl sulphate surfactant. Taurate surfactants useful in the present invention are salts of fatty acid amides of N-methyl taurine. They conform generally to the structural formula:

RC(O)N(CH₃)CH₂CH₂SO₃M

Where RC(O)- represents a fatty acid radical and M represents sodium, potassium, ammonium or triethanolamine. Fatty acids having carbon chain lengths of from 10 to 20, including those derived from coconut, palm and tall oil are used. In one embodiment the fatty acid is derived from coconut. In one embodiment, sodium salts are used. In one embodiment the taurate is sodium methyl cocyl taurate. This taurate surfactant is sold under the trademark by Adinol CT by Croda.

The taurate surfactant may be present in an amount from about 0.1 to about 10% of the non-aqueous composition. In one embodiment the taurate surfactant is present in an amount from about 0.1 to about 5% by weight of the non aqueous composition. In one embodiment the taurate surfactant is present in an amount from about 0.5 to about 2.0% by weight of the non-aqueous composition.

Alkyl sulphate surfactants of use in the invention have the following structural formula:

R¹OSO₃M

R¹ represents a fatty alcohol moiety and M represents sodium, potassium, ammonium or triethanolamine. Fatty alcohols having carbon chain lengths of from about 10 to about 20, including those derived from coconut, palm oil and tall oil. In one embodiment, the fatty alcohol is lauryl alcohol. In one embodiment, a sodium salt is used. In one embodiment the alkyl sulphate is sodium lauryl sulphate.

The alkyl sulphate surfactant may be present in an amount from about 0.1 to about 10% of the non-aqueous composition. In one embodiment the alkyl sulphate surfactant may be present in an amount from about 0.1 to about 5% by weight of the non aqueous composition. In one embodiment the alkyl sulphate surfactant is present in an amount from about 0.5 to about 2.0% by weight of the non-aqueous composition.

In certain embodiments, the surfactant system consists of a first surfactant which is a betaine, and a second surfactant which consists of a mixture of a taurate and a C₁₀₋₂₀ alkyl sulphate surfactant as hereinabove described. In one embodiment the surfactant system consists of a first surfactant which is a betaine and second surfactant which consists of a mixture of sodium methyl cocyl taurate and sodium lauryl sulphate.

The composions of the present invention comprise hyaluaronic acid or a salt thereof.

Hyaluronic acid, (often abbreviated as HA -conjugate base hyaluronate, also called hyaluronan, is an anionic, nonsulfated glycosaminoglycan distributed widely throughout connective, epithelial, and neural tissues.

In certain embodiments the hyalauronic acid is present in salt form. Particularly preferred salts include sodium hyalurate and potassium hyalurate.

Preferably the composition comprise between 0.01% and 1 % by weight of hyalauronic acid or salt thereof. More preferebaly between 0.05 % and 0.25 % by weight of hyalauronic acid or salt thereof.

In one embodiment of the invention, a dentifrice composition is prepared comprising the following components in percent by weight:

| | |
|---|---|
| Hyalauronic acid or salt thereof | about 0.01 to about 1 |
| Betaine | about 0.05 to about 4 |
| Taurate | about 0.5 to about 2 |
| Carbomer | about 0.3 to about 1 |
| Glycerin | about 50 to about 70 |
| Polyethylene glycol | about 15 to about 25 |

In one embodiment of the invention, a dentifrice composition is prepared comprising the following components in percent by weight:

| | |
|---|---|
| Hyalauronic acid or salt thereof | about 0.01 to about 1 |
| Betaine | about 0.05 to about 4 |
| SLS | about 0.5 to about 2 |
| Carbomer | about 0.3 to about 1 |
| Glycerin | about 50 to about 70 |
| Polyethylene glycol | about 15 to about 25 |

The compositions of the present invention may further comprise other ingredients.

In one aspect a dentifrice composition according to the invention may comprise a dentifrice additive that is unstable or incompatible with an aqueous environment.

An example of such an additive is a bioactive glass of the type disclosed in WO96/10985, WO 97/27158 and WO 99/13852. In an aqueous environment such a bioactive glass releases ions causing a significant increase in pH which can adversely affect the stability (especially upon long-term storage) of any excipients contained within the dentifrice. Formulating a bioactive silica-based glass in the non-aqueous dentifrice of the present invention prevents the release of ions within the dentifrice thereby controlling pH and increasing long-term storage stability of the dentifrice.

In one embodiment the bioactive glass for use in the invention has a composition consisting of about 45% by weight silicon dioxide, about 24.5% by weight sodium oxide, about 6% by weight phosphorus oxide, and about 24.5% by weight calcium oxide. One such bioactive glass is available commercially under the trade name, NovaMin^{®}, also known as 45S5 Bioglass^{®}.

The bioactive glass is present in an amount ranging from about 1 to about 20% by weight of the non-aqueous composition. In one embodiment, the bioactive glass is present in an amount from about 1 to about 15% by weight of the non-aqueous composition. In an alternative embodiment, the bioactive glass in the non-aqueous composition is present in an amount from about 1 to about 10% by weight of the non-aqueous composition. In a further alternative embodiment the bioactive glass is present in an amount from about 2 to about 8 % by weight of the non-aqueous composition.

The composition comprisies a carboxyvinyl polymer as a thickening agent. Howwcwe a composition according to the invention may further comprise an additional thicking agent as required. A suitable example of a further thickening agent is an inorganic thickening agent such as a thickening silica.

An example of a thickening silica, for example, is a colloidal hydrated silica, available commercially for example as Sident 22S or Syloid 244FP.

In one embodiment the thickening silica is present in the range of from about 0 to about 15%, suitably from about 5.0 to about 15.0% by weight of the non-aqueous composition.

A dentally acceptable abrasive may optionally be added to the non-aqueous composition. Advantageously, the presence or absence of a dentally acceptable abrasive as well as the amount of such abrasive may be used to selectively control the abrasivity of the dentifrice composition made with the non-aqueous compositions of the invention. By way of example, and if present, the bioactive glass may provide an acceptable amount of abrasivity for the non-aqueous composition depending upon the ultimate use. By further way of example, a desired amount of dentally acceptable abrasive may be added to increase the abrasivity of the overall non-aqueous composition.

Suitable abrasives for use in the non-aqueous composition include, for example, amorphous, gelled, precipitated or fumed silica, zinc orthophosphate, sodium bicarbonate (baking soda), plastic particles, alumina, hydrated alumina, calcium carbonate, calcium pyrophosphate, insoluble metaphosphates or mixtures thereof.

The silica abrasive may be a natural amorphous silica, for instance diatomaceous earth; or a synthetic amorphous silica such as a precipitated silica. By way of example, silica abrasives include those marketed under the following trade names Zeodent, Sident, Sorbosil or Tixosil by Huber, Degussa, Ineos and Rhodia respectively.

Suitably a silica abrasive is present in an amount up to 25% by weight of the total composition, for example from 1 to 20% by weight for example from 5 to 15% by weight of the total composition.

Generally, an amount of abrasive suitable for use in the non-aqueous composition of the present invention will be empirically determined to provide an acceptable level of cleaning and polishing, in accordance with the techniques well known in the art. Suitably, the abrasive is present in an amount from about 0 to about 60%, typically from about 5 to about 30%, by weight of the non-aqueous composition. The amount depending on the abrasive character of the particular silica and the level desired in the end product.

The non-aqueous compositions of the invention may additionally optionally contain one or more oral care active agents conventionally used in dentifrice formulations. Such agents may include, by way of example, a fluoride source, a desensitizing agent, an anti-calculus agent, an anti-erosion agent, an antimicrobial agent, an anti-plaque agent, a whitening agent, an oral malodour agent or a mixture of at least two thereof.

Suitable sources of fluoride ions for use in the compositions of the present invention include an alkali metal fluoride such as sodium fluoride, an alkali metal monofluorophosphate such a sodium monofluorophosphate, stannous fluoride, or an amine fluoride in an amount to provide from 25 to 3500pm of fluoride ions, preferably from 100 to 1500ppm. The level of fluoride allowed in commercial dentifrices is heavily dependent on local laws, 900 ppm , 1100 ppm and 1500 ppm levels being most common.

In addition to or as an alternative to the bioactive glass, a further desensitizing agent, including a tubule blocking agent or a nerve desensitizing agent and mixtures thereof, for example as described in WO 02/15809 (Block) may be included in a composition according to the invention. Such further optional desensitizing agent(s) include a strontium salt such as strontium chloride, strontium acetate or strontium nitrate or a potassium salt such as potassium citrate, potassium chloride, potassium bicarbonate, potassium gluconate and especially potassium nitrate.

Polyphosphates are known to help retard calculus formation and are examples of anticalculus agents suitable for use in the invention. A polyphosphate is generally understood to consist of two or more phosphate groups arranged primarily in a linear configuration, although some cyclic derivatives may be present. Polyphosphates of use in the invention include pyrophosphates, polyphosphates having three or more polyphosphate groups such as sodium tripolyphosphate, and polyphosphates having four or more polyphosphate groups such as tetrapolyphosphate and hexametaphosphate among others.

Compositions of the invention may further comprise an antierosion agent, for example a polymeric mineral surface active agent as described in WO 04/054529 (Procter & Gamble).

Compositions of the present invention will contain additional formulating agents such as flavouring agents, sweetening agents, opacifying or colouring agents and preservatives, selected from those conventionally used in an oral hygiene composition art for such purposes.

In general, the optional agents may be used in a minor amount or proportion of the overall formulation. By way of example, such components are usually present in from about 0.001 to about 5% by weight of the non-aqueous composition.

The dentifrice composition typically has a viscosity suitable for application to the oral cavity. The viscosity will vary depending on the type of dentifrice composition made and the ultimate use thereof. One of skill in the art can readily prepare compositions with suitable viscosities for use in the oral cavity from the teachings provided herein.

The compositions according to the present invention may be prepared by admixing the ingredients in the appropriate relative amounts in any order that is convenient.

The invention is further illustrated by the following Examples and experiments

### Formula 1 - Non-aqueous Composition (control)

| Ingredient | % w/w |
|---|---|
| Glycerol | 59.33 |
| Polyethylene Glycol 400 | 20.00 |
| Silicon Dioxide | 8.00 |
| Sodium Tripolyphosphate | 5.00 |
| Cocamidopropyl Betaine | 0.36 |
| Sodium Lauryl Sulphate | 1.10 |
| Stannous Fluoride | 0.454 |
| Carbomer Homopolymer | 0.80 |
| Minor ingredients | ~5.0 |

### Formula 2 -

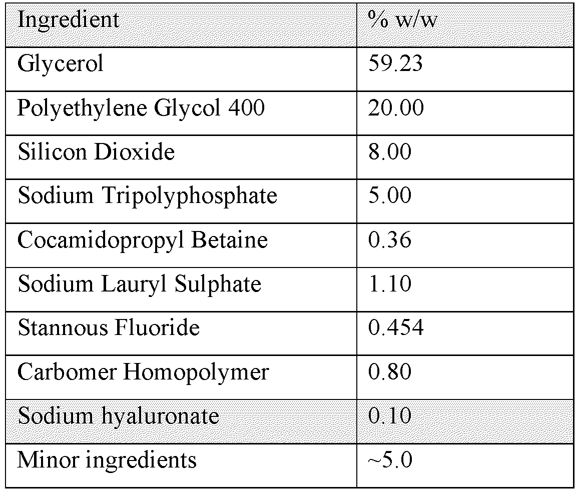

### Formula 3 -

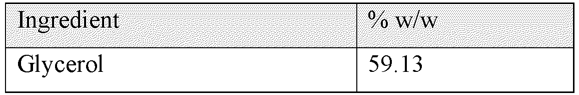

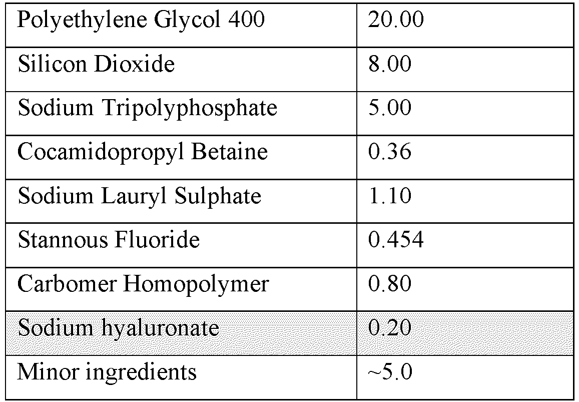

### Rheology data:

Formulas 1 and 2 were tested for their rheological response to shear.
A 1:3 paste to water slurry was made of each formula and these slurries were mixed for ten minutes and then tested.

### Equipment Used:

Malvern Kinexus:2T32116
CP2/60 - Top Geometry
PLS61 S3100 SS - base plate
Method: shear rate table MW
Start shear rate: 5s-1 up to 500s-1
samples per decade: 10.
Manual gap > 0.07mm.

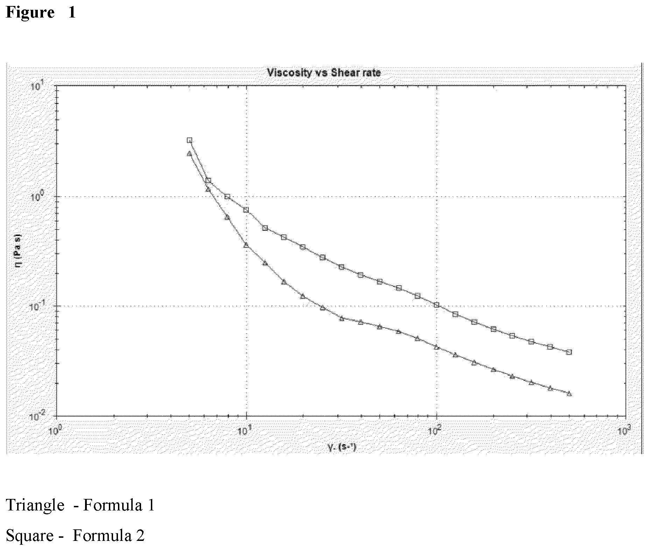

After a ten minute mix with water, the paste that contains HA (Formula 2) is shown in the graph (Figure 1) above to have a higher viscosity than the paste that does not contain HA (Formula 1) . Both pastes show a similar decrease in viscosity as more shear is applied to them, however the paste derived from formula 2 does not have as steep a curve, indicating it may be slightly more robust.

The formulations were then tested for senory effects.

### Study Objectives

A blind sensory study was conducted with Haleon OH Expert panel to determine if the addition of 0.1%w/w (formula 2) and 0.2% w/w (formula 3) sodium hyaluronate) significantly improve the organoleptic profile of the toothpaste vs a control (Formula 1).

### Sensory Method

Descriptive Profiling: Foam, flavour, aftertaste and mouthfeel characteristics of the formulations were recorded on a 0-100 scale. Each sample was evaluated in triplicate. ANOVA was applied at α = 5% to determine significant differences between samples.

### Observations:

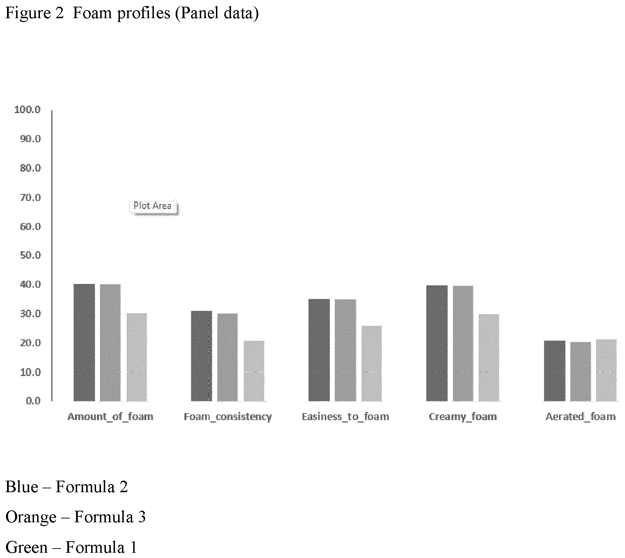

Sodium hyaluronate significantly (p<0.01) improved the foam profile of a non aqueous dentifrice composition. The formulations with sodium hyaluronate elicited: more foam, thicker and more creamy which lathered faster vs. the control. There was no concentration effect (Figs. 2).

The addition of sodium hyaluronate gave significantly (p<0.01) to the toothpaste a texture more dense, resulting to a paste slightly more difficult to break during brushing vs. control There were only marginal effects of sodium hyaluronate on other mouthfeel attributes such as stickiness and mouth coating.

The addition of sodium hyaluronate significantly (p<0.01) slightly decreased the sweetness and increased a lingering bitterness, with the 0.2% SH formulation more bitter.

The freshness sensation was significantly (p<0.01) slightly more lingering for the formulations with 0.1% w/w sodium hyaluronate (example 2) and 0.2% w/w sodium hyaluronate (example 3) vs. control (formula 1).

### Conclusions

The addition of sodium hyaluronate significantly (p<0.01) improved the foam profile of The formulas. The formulations 2 and 3 provided:
- more foam
- a thicker foam
- a creamier foam
which lathered faster vs. control formula 1

There was no concentration effect of the NaHA. Formla 2 and 3 performed equally in the tests.

Formulas 2 and 3 offered bother sensory and rheological improvements over the control formulation.

## Claims

1. A non-aqueous dentifrice composition comprising;
a) a solvent, wherein the solvent is selected from the group comprising glycerin, sorbitol, propylene glycol, polyethylene glycol and mixtures thereof.
b) a caboxyvinyl polymer wherein the carboxyvinyl polymer is present in the range from 0.1 to 7.5% by weight of composition;
c) a surfactant system consisting of a first surfactant which is a betaine in combination with a second surfactant which is a taurate or a C₁₀₋₂₀ alkyl sulphate or mixtures thereof; and
d) hyaluronic acid or a salt thereof.

2. The non-aqueous dentifrice composition according to claim 1 wherein the solvent comprises glycerin.

3. The non-aqueous dentifrice composition according to claim 1 or claim 2 wherein the glycerin is present in an amount ranging from 35 to 75% by weight of the composition.

4. The non-aqueous dentifrice composition according to any one of the previous claims wherein the solvent comprises polyethylene glycol.

5. The non-aqueous dentifrice composition according to claim 4 wherein the polyethylene glycol is present in an amount ranging from 15 to 25% by weight of the non-aqueous composition.

6. The non-aqueous dentifrice composition according to any of the previous claims wherein the betaine is cocoamidopropyl betaine.

7. The non-aqueous dentifrice composition according to any of the previous claims wherein the betaine is present in an amount from 0.05 % to about 4 % such as from about 0.2 % to about 2 % by weight of the composition.

8. The non-aqueous dentifrice composition according to any of the previous claims wherein the second surfactant comprises a taurate.

9. The non-aqueous dentifrice composition according to claim 8 wherein the taurate is the sole second surfactant.

10. The non-aqueous dentifrice composition composition according to any one of claims 8 or 9 wherein the taurate is sodium methyl cocyl taurate.

11. The non-aqueous dentifrice composition according to any one of claims 8 to 10 wherein the taurate is present in an amount from 0.5 % to 2% by weight of the composition.

12. The non-aqueous dentifrice composition according to any one of claims 1 to 7 wherein the second surfactant comprises a C₁₀₋₂₀ alkyl sulphate.

13. The non-aqueous dentifrice composition according to claim 12 wherein the C₁₀₋₂₀ alkyl sulphate is the sole second surfactant.

14. The non-aqueous dentifrice composition according to any one of claims 1 to 8 and 12 or 13 wherein the C₁₀₋₂₀ alkyl sulphate is sodium lauryl sulphate.

15. The non-aqueous dentifrice composition according any of the previous claims wherein the hyalauronic acid or a salt thereof comprises between 0.01 % and 1 % by weight of the dentifrice composition.

16. The non-aqueous dentifrice composition according to claim 15 wherein the hyalauronic acid or a salt thereof comprises between 0.05 % and 0.25 % by weight of the dentifrice composition.

17. The non-aqueous dentifrice composition according to any of the previous claims wherein the hyalauronic acid or a salt thereof comprises sodium hyaluronate.

18. The non-aqueous dentifrice composition according to any one of claims 1 to 17 comprising an oral care active agent selected from a fluoride source, a desensitizing agent, an anti-calculus agent, an anti-erosion agent, an antimicrobial agent, an anti-plaque agent, a whitening agent, an oral malodour agent or a mixture of at least two thereof.
